# EUROPEAN PATENT SPECIFICATION

(11) **EP 2 107 941 B1**
(45) Date of publication and mention of the grant of the patent: **03.10.2018**
(21) Application number: 08701970.9
(22) Date of filing: 29.01.2008
(51) Int. Cl.: B01J 37/08, B01J 23/75, B01J 23/889, C10G 2/00

(54) **PREPARATION OF FISCHER-TROPSCH CATALYSTS**
HERSTELLUNG EINES FISCHER-TROPSCH-KATALYSATORS
PRÉPARATION DE CATALYSEURS DE FISCHER-TROPSCH

(30) Priority: 30.01.2007 GB 0701740
(43) Date of publication of application: 14.10.2009
(73) Proprietor: GTL. F1 AG, 8032 Zurich (CH); Johnson Matthey PLC, London EC4A 4AB (GB)
(72) Inventor: ERI, Sigrid, N-7053 Ranheim (NO); RYTTER, Erling, N-7049 Trondheim (NO); BORG, Øyvind, N-7051 Trondheim (NO); ANTONINI, Alejandro, Martin, Stockton on Tees TS17 0YW (GB); MCCULLOCH, Kate, Elizabeth, Cleveland TS14 7PT (GB)
(74) Representative: Copsey, Timothy Graham
(86) International application number: PCT/GB2008/000300
(87) International publication number: WO 2008/093073

(56) References cited:
- WO-A-99/61143
- GB-A- 570 105
- GB-A- 1 183 201
- US-A- 2 987 487
- US-A- 3 141 742
- US-A- 4 729 981
- BETANCOURT P ET AL: "A study of the ruthenium-alumina system" APPLIED CATALYSIS A: GENERAL, ELSEVIER SCIENCE, AMSTERDAM, NL, vol. 170, no. 2, 15 June 1998 (1998-06-15), pages 307-314, XP004271457 ISSN: 0926-860X
- VAN DE LOOSDRECHT J ET AL: "Calcination of Co-Based Fischer-Tropsch Synthesis Catalysts" TOPICS IN CATALYSIS, KLUWER ACADEMIC PUBLISHERS-PLENUM PUBLISHERS, NE, vol. 26, no. 1-4, 1 December 2003 (2003-12-01), pages 121-127, XP019292006 ISSN: 1572-9028 cited in the application
- ØYVIND BORG ET AL.: "Effect of calcination atmosphere and temperature on ?-Al2O3 supported cobalt Fischer-Tropsch catalysts" TOPICS IN CATALYSIS, [Online] vol. 45, no. 1-4, August 2007 (2007-08), pages 39-43, XP002474070 Retrieved from the Internet: URL:http://www.springerlink.com/content/q3 7u8x8583661441/fulltext.pdf> [retrieved on 2008-03-14]

## Description

The present invention is concerned with the preparation of Fischer-Tropsch catalysts. The Fischer-Tropsch (FT) reaction for conversion of synthesis gas, a mixture of CO and hydrogen, possibly also containing essentially inert components like CO₂, nitrogen and methane, is commercially operated over catalysts containing the active metals iron (Fe) or cobalt (Co). However, the iron catalysts exhibit a significant shift reaction, producing more hydrogen in addition to CO₂ from CO and steam. Therefore the iron catalyst will be most suited for synthesis gas with low H₂/CO ratios (< 1.2), *e.g.* from coal or other heavy hydrocarbon feedstock, where the ratio is considerably lower than the consumption ratio of the FT reaction (2.0-2.1).
In systems where the H₂/CO ratio in the synthesis gas is higher, e.g. where the hydrocarbon feedstock is methane, cobalt is the preferred catalyst. The present invention is particularly concerned with Co based catalysts.
Normally, the active FT metal is dispersed on a solid, porous support. In this way, a large portion of the Co is exposed as surface atoms where the reaction can take place. The support can be alumina, titania or silica, but in fact, other oxides such as zirconia, magnesia, zeolites have been used, as has carbon.
To enhance the catalyst performance, e.g. by facilitating the reduction of cobalt oxide to cobalt metal prior to the FT synthesis, it is common to add different promoters. In this regard, rhenium, ruthenium, platinum, iridium and other transition metals can all be beneficial.

A number of different impregnation procedures are known in the art which use various solvents and chemicals, and which are suitable. However, in the present specification, the examples involve melt impregnation or incipient wetness impregnation of cobalt nitrate hexahydrate (Co(NO₃)₂.6H₂O) onto the support. It is well known that the impregnation method may influence the dispersion of the active metal (cobalt) and hence the catalytic activity. Generally, after impregnation, the impregnated catalyst is dried, typically at 80-120°C, to remove water from the catalyst pores, and then calcined typically at 200-450°C, e.g. at 300°C for 2-16 h.

The conditions during calcination may also have a significant effect on the final catalyst properties. According to EP-A-0 421 502, the activity may be improved, if during calcination, the catalyst is exposed to an atmosphere containing large amounts of nitrogen oxides. In contrast, however, van de Loosdrecht et al. (Topics in Catalysis, Vol. 26, 2003, pp. 121-127) recorded high cobalt metal surface areas and high catalytic activities when the concentration of nitrogen oxides and water was kept low during calcination.

US4,279,981 to Kobylinksi, T.P. et al relates to an ROR catalyst for synthesis gas conversion to liquid hydrocarbons. WO99/61143 to Conoco Inc. discloses a process for producing hydrocarbons. The process involves contacting a feed stream with a catalyst to produce hydrocarbons, where the catalyst includes at least one catalytic metal.

Betancourt P. et al discusses the use of ruthenium catalysts in "A study of the ruthenium-alumina system", published in Applied Catalysis A: General, Elsevier Science, Amsterdam, NL, vol. 170, no.2, 15 June 1998, pages 307-314, XP004271457, ISSN: 1572-9028.

US3,141,742 to Dye, R.F. et al relates to a process for activating a solid catalyst in a fluidized bed. US2,987,487 to Stevens, J.I. et al describes a process and apparatus for the activation of catalysts, including catalysts which are water sensitive.
It is an object of the present invention to provide an improved F-T catalyst. According to the invention, there is provided a method of producing a catalyst for use in a Fischer-Tropsch synthesis reaction, comprising the steps of: impregnating a catalyst support material with an active cobalt catalyst component to form a catalyst precursor; and calcining and subsequently reducing the catalyst precursor, wherein the catalyst precursor is calcined in an atmosphere of a dry calcining gas at a temperature in the range of 150°C to 450°C for a period of 1 to 16 hours and at a flow rate giving a gas hourly space velocity of greater than 1000hr⁻¹, the water content of the calcining gas being reduced in a gas drying apparatus by one or more of the steps of condensing, sorption, membrane separation and pressure reduction to produce a vapour pressure <1933.2 Pa (<14.5 mm Hg) and wherein after calcination and reduction, the specific surface area of the cobalt in the catalyst is in the range of 5 to 30 m2/g.

Preferably, the calcining step is carried out by passing the dry calcining gas through or over a bed of catalyst precursor particles and preferably, the active metal-based catalyst component is cobalt. The dry calcining gas may be an inert gas, such as nitrogen, or an oxygen containing gas such as air. Preferably, the impregnation step includes impregnation with a catalyst promoter component, preferably a rhenium component and the catalyst, after calcination and reduction, contains an amount of rhenium in the range 0.1 to 2 wt% as a promoter, assuming complete reduction of rhenium.

Preferably, the precursor is subjected to a drying step, prior to the calcining step. Preferably, the drying step is carried out at a temperature in the range 80 to 160°C, preferably 110 to 150°C, for a period of 0.2 to 10 hours, preferably 0.5 to 4 hours. The catalyst is subjected to a reduction step after the calcining step. Preferably, the catalyst has a metal surface area that is increased by at least 10% as compared to a catalyst prepared by calcinations in air without water vapour pressure reduction.

Preferably, the impregnating step is achieved by means of melt impregnation or incipient wetness impregnation techniques. Other suitable impregnation techniques include: precipitation from solution; precipitation/deposition; chemical vapour deposition; and vacuum impregnation. Preferably, the support material is impregnated with cobalt nitrate hexahydrate (Co(NO₃)₂.6H₂O). Preferably, the support material is impregnated with an aqueous solution of cobalt nitrate hexahydrate, containing an amount of cobalt to give a catalyst with a cobalt loading in the range 10 to 50 wt%, preferably 10 to 25 wt%, assuming complete reduction of cobalt. Preferably, the aqueous solution also contains an amount of rhenium to give a catalyst with a rhenium loading in the range 0.1 to 2 wt%, preferably 0.2 to 1 wt%, as a promoter, assuming complete reduction of rhenium.

Preferably, the support material is alumina, titania, silica, zirconia, magnesia or zeolite. The most preferred material is γ-alumina, though any other form of alumina alone or in combination with a spinel-type aluminate, e.g. α-alumina together with nickel aluminate, may be used.

Preferably, the support material contains at least 10% by weight of a spinel compound composed of a divalent metal and aluminium. Preferably, the divalent metal is nickel or zinc. Preferably, the support material, prior to impregnation, has a specific surface area in the range 20 to 500 m²/g, preferably 30 to 200 m²/g. Preferably, the support material, prior to impregnation, has a pore volume greater than 0.1 cm³/g, preferably greater than 0.2 cm³/g.

We have found, surprisingly, that the humidity of the gas to which the material is exposed during calcination can have an effect of the F-T activity of the resulting catalyst. Hence, we have found that exposure of the catalyst precursor to dry gases, i.e. gases in which the water content has been reduced, during the calcination step, offers improved Fisher-Tropsch catalysts. The gas used during calcination may be an oxygen-containing gas, e.g. air, or an inert gas such as nitrogen, or mixtures of these. Air is preferred.

The calcining step is conducted at a temperature in the range 150 to 450°C, more preferably 200 to 350°C, for a period of 1 to 16 hours, more preferably 1 to 10 hours. In the present invention the water content of the gas used in the calcination step is reduced by one or more steps of condensing, sorption, membrane separation, or pressure reduction, to have a water vapour pressure preferably <1933.2Pa (<14.5mm Hg). More preferably, the gas passed over the catalyst precursor during the calcining step has a humidity defined by a water vapour pressure of <666.7Pa (< 5 mm Hg), most preferably <133.3Pa (< 1 mm Hg), providing a dew point of < 1.3°C, more preferably < -17.3°C respectively.

The term "passed over the catalyst precursor" means that the gas is passed over the individual particles of the precursor. Conveniently, this is achieved by passing the gas through a bed of the precursor particles. The gas may be preheated prior to being passed through the precursor particles. In a preferred embodiment, the calcination is performed on a fluidised bed of catalyst precursor in fluidised bed calciner.
Preferably, the dry calcining gas contains less than 500 vppm of water. Thus, the calcining gas may contain, in the range of 20 to 1000 vppm of water, preferably between 50 and 500 vppm of water. The dry calcining gas may be obtained by cooling the gas thereby condensing water or by using a sorption material to remove water. The reduced water vapour pressure in the calcining gas may be obtained by mixing an un-dried gas with a stream of dried gas, by compressing the gas to condense water and separating the condensed water, by passing the gas through a membrane that physically separates the water from the gas, or by performing the calcination under vacuum, preferably at a pressure of less than 20.3kPa (0.2 atm).
Preferably, the gas is passed through a bed of catalyst precursor particles. The gas a flow rate giving a gas hourly space velocity (GHSV) >1000 hr⁻¹.

The surface area of the cobalt in the catalyst, after calcination and reduction is in the range 5 to 30 m²/g catalyst, preferably 10 to 25 m²/g.

The description also extends to a catalyst produced by the above method. Preferably, the cobalt content of the catalyst is from 10 to 40% by weight, preferably from 10 to 25% by weight.

The description also extends to the use of such a catalyst in an F-T synthesis reaction. Preferably, the reaction is carried out in a slurry bubble column reactor. Preferably, H₂ and CO are supplied to a slurry in the reactor, the slurry comprising the catalyst in suspension in a liquid including the reaction products of the H₂ and CO, the catalyst being maintained in suspension in the slurry at least partly by the motion of the gas supplied to the slurry.

The description also extends to a process for the production of hydrocarbons which comprise subjecting H₂ and CO gases to a Fischer-Tropsch synthesis reaction in a reactor in the presence of the catalyst.
Preferably, the reaction is a three-phase reaction in which the reactants are gaseous, the product is at least partially liquid and the catalyst is solid. Preferably, the reaction is carried out in a slurry bubble column reactor. Preferably, the H₂ and CO are supplied to a slurry in the reactor, the slurry comprising the catalyst in suspension in a liquid including the reaction products of the H₂ and CO, the catalyst being maintained in suspension in the slurry at least partly by the motion of the gas supplied to the slurry.
Preferably the reaction temperature is in the range 190 to 260°C, preferably 210 to 240°C, and the pressure is in the range 10 to 60 bar, preferably 15 to 35 bar. Preferably, the ratio H₂/CO of the gases supplied to the Fischer-Tropsch synthesis reactor is in the range 1.1 to 2.2, preferably 1.5 to 1.95, and the superficial gas velocity in the reactor is in the range 5 to 60 cm/s, preferably 20 to 40 cm/s.

Preferably, the product of the Fischer-Tropsch synthesis reaction is subsequently subjected to post-processing. The post-processing may include de-waxing, hydro-isomerisation, hydro-cracking and combinations of these.

An apparatus for carrying out the method of the invention is described, comprising a calcination vessel through which the catalyst precursor and calciner gas may pass having a catalyst precursor inlet, a calcined catalyst precursor outlet, a calciner gas inlet and a calciner gas outlet, and in which the calciner gas inlet is operatively connected to gas drying apparatus. Preferably, the gas drying means comprises condensing means that cool the gas to below the dew point of the water contained therein to condense and optionally to freeze the water and thereby separate the condensed or frozen water from the air fed to the calcination. Alternatively, the gas drying apparatus comprises a vessel having a wet gas inlet and a dry gas outlet and an absorption material, preferably a particulate zeolite, disposed between the wet gas inlet and the dry gas outlet.

Porous catalyst support materials typically have specific surface areas between 30 and 500 m²/g. These supports can be prepared by spray drying techniques of an appropriate solution in order to obtain essentially spherical particles of appropriate size, *e.g.* 80% in the range between 30-150 µm. After spray-drying, the material is calcined at a high temperature to give the appropriate support crystal size and pore structure.

It is important that the total pore volume of the support is sufficiently high, above 0.1 cm³/g or better, above 0.2 cm³/g, or even better above 0.6 cm³/g. The pore volume is often measured by nitrogen adsorption/desorption. This method does not take into account large pores where a mercury porosimeter is more relevant. A less accurate, but more practical parameter is the measured water absorptivity, which can be directly correlated with the amount of cobalt that can be impregnated on the catalyst by the incipient wetness procedure. A high pore volume will give a light material suitable for operation in a slurry environment and ease the impregnation by minimising the number of impregnation steps required. At the same time the support, and the final catalyst, should have sufficient strength for extended operation of months and years with minimal attrition of the materials. This can be tested in a slurry environment or by the ASTM method applicable for testing FCC (fluid catalytic cracking) catalysts.

The invention may be carried into practice in various ways and some embodiments will now be specifically described in the following non-limiting examples.

The starting alumina material used for all catalysts in the present invention was Puralox SCCa γ-Al₂O₃ from Sasol.

### EXAMPLE 1 - Effect of Air Circulation

A catalyst precursor was prepared by one-step incipient wetness impregnation of γ-Al₂O₃ (BET surface area = 187 m²/g, Pore volume = 0.73 cm³/g) with an aqueous solution of cobalt nitrate hexahydrate. The sample contained a nominal amount of 20 wt% cobalt and 1 wt% rhenium, as calculated assuming reduced catalysts with complete reduction of cobalt. The actual metal loading as determined by XRF (x-ray fluorescent spectroscopy) or ICP (inductively coupled plasm spectrometry) may vary up 10%, *e.g.* for a catalyst with nominal loading of 20 wt%, the actual amount cobalt can vary between 18 and 22 wt% of the total reduced catalyst weight. The effect of air humidity for the catalytic properties was tested by varying the calcination arrangement. One (sample 1) was calcined in a crucible located inside an oven. The air flow was not passed directly through the sample. The other sample (sample 2) was calcined as a bed of catalyst particles inside a glass reactor located inside the same oven and subjected to a continuous flow of air. In this case, the air flowed directly through the sample. The calcination temperature and time were 300°C and 10 h, respectively. For Sample 1, ambient air surrounded the crucible before heating, whereas dry instrument air at room temperature was passed via a tube through the wall of the oven for Sample 2. In the latter case, the water content of the air was 500 vppm.

The catalytic properties of sample 1 and 2 are given in Table 1. The high activity of sample 2 compared to sample 1 was related to the lower amounts of water and NO*ₓ* in the calcination atmosphere for the latter sample. By passing dry air directly through the sample, we believe that the calcination decomposition products were removed very efficiently. The calcination effect is probably related to different cobalt surface areas after calcination since it is generally believed that the FT-reaction is non-structure sensitive.

**Table 1. Catalytic properties of catalysts**

| *Catalyst* | *Relative activity* | *Relative selectivity* |
|---|---|---|
| 1 | 1.53 | 0.908 |
| 2 | 1.91 | 0.909 |

### EXAMPLE 2 - Effect of Air Humidity

The effect of air humidity for the cobalt surface area was tested for another series of catalysts. A catalyst precursor was prepared by one-step incipient wetness impregnation of a γ-Al₂O₃ support (of the same kind as used in Example 1) with an aqueous solution of cobalt nitrate hexahydrate. The sample contained a nominal amount of 20 wt% cobalt. After impregnation, the catalyst precursor was dried at 110°C for 3 h and subsequently split into samples of 1.2 g. These samples were calcined for 10 h under different conditions (air at 30 ml/min; air at 50 ml/min; 50% air/50% steam at 30 ml (min) and at different temperatures. The water content in the air was < 40 vppm.

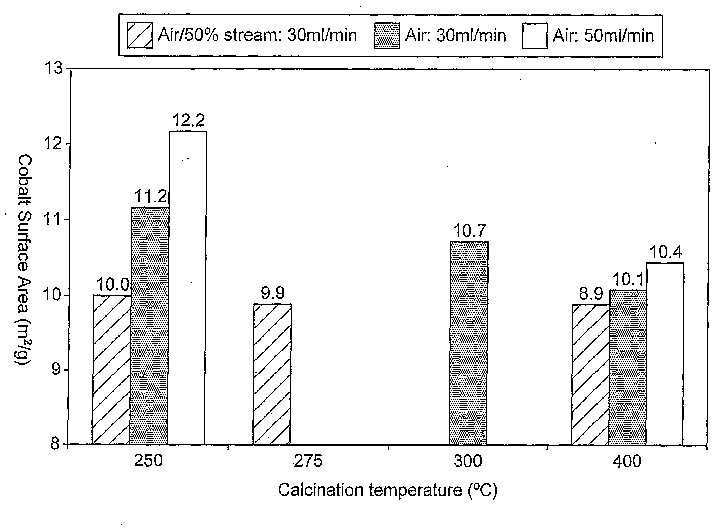

The cobalt surface areas obtained for the catalysts of this series are given in Figure 1. Calcination in humid air clearly resulted in lower cobalt surface area than calcination in dry air. Increasing the air flow rate and therefore decreasing the water content during calcinations is clearly beneficial. The difference was largest for the lowest calcination temperatures. The reason for the big difference was probably different Co₃O₄ crystallite sizes prior to *in situ* reduction. X-ray diffraction data gave a crystallite size of 13.2 and 6 nm, respectively.

### EXAMPLE 3 - Effect of Air Humidity

The catalysts of this series contain a nominal amount of 20wt% Co and 0.5 wt% Re. Cobalt nitrate hexahydrate was heated above the melting point and mixed with ammonium perrhenate. The mixture was then added to the support. Before impregnation, the catalyst support (BET surface area = 175 m2/g, Pore volume = 0.71 cm3/g) was pre-calcined at 500°C.

The freshly prepared catalysts were dried at a temperature of 110°C. After impregnation and drying, calcination was carried out in a fluidised bed reactor. In all cases, the calcination temperature and time were 250°C and 2 hours, respectively. The effect of humidity during calcination was studied by feeding air flows of different humidity to the calcination reactor. Both dry instrument air (dew point < -30°C) and air with higher levels of humidity were used as calcination agents. The water content (and air dew point) was controlled by saturating air with water in a heated bubbler and mixing with dry air. In comparison to the present invention illustrated by experiments A, B, C & D, experiments E, F & G were run with humidity's above 1933.2 Pa (14.5 mmHg). The prepared catalysts are summarised in Table 2, which clearly shows that the air humidity level is important for the catalytic activity. Fisher-Tropsch activities and selectivity's were measured relative to experiment G. Calcination in air with a dew point of 0°C or below resulted in relative activities in the range 1.10 to 1.14 in the Fisher-Tropsch synthesis. The activities after calcination in humid air were lower when the inlet air dew point was 17°C or higher.

**Table 2. Experimental conditions and catalytic data.**

| Catalyst name | Catalyst composition | | Calcination conditions | | Catalytic performance | |
|---|---|---|---|---|---|---|
| | Co (wt%) | Re (wt%) | Dew point air (°C) | Space velocity Litre/gCo/hr | Relative activity | Relative C5+ selectivity |
| A | 20 | 0.5 | -30 | 22.4 | 1.14 | 1.01 |
| B | 20 | 0.5 | -30 | 22.5 | 1.10 | 1.02 |
| C | 20 | 0.5 | 0 | 20.3 | 1.12 | 1.01 |
| D | 20 | 0.5 | 5 | 20.3 | 1.04 | 1.01 |
| E | 20 | 0.5 | 17 | 22.5 | 0.99 | 1.01 |
| F | 20 | 0.5 | 17 | 22.5 | 0.98 | 1.00 |
| G | 20 | 0.5 | 30 | 23.9 | 1.00 | 1.00 |

### Hydrogen chemisorption

Hydrogen adsorption isotherms were recorded on a Micromeritics ASAP 2010 unit at 313 K. The samples were reduced *in situ* in flowing hydrogen at 623 K for 16 h. The temperature was increased by 1 K/min from ambient to 623 K. After reduction, the samples were evacuated at 623 K and cooled down under vacuum to 313 K. An adsorption isotherm was recorded in the pressure interval between approximately 2.6kPa to 68kPa (20 to 510 mmHg). The amount of chemisorbed hydrogen was calculated by extrapolating the linear part of the isotherm to zero pressure. In order to calculate the cobalt surface area, it was assumed that two cobalt sites were covered by one hydrogen molecule and that the area of one cobalt atom is 6.62 · 10⁻²² m²/atom.

## Claims

1. A method of producing a catalyst for use in a Fischer-Tropsch synthesis reaction, comprising the steps of: impregnating a catalyst support material with an active cobalt catalyst component to form a catalyst precursor, and calcining and subsequently reducing the catalyst precursor, **characterised in that** the catalyst precursor is calcined in an atmosphere of a dry calcining gas at a temperature in the range of 150°C to 450°C for a period of 1 to 16 hours and at a flow rate giving a gas hourly space velocity of greater than 1000hr⁻¹, the water content of the calcining gas being reduced in a gas drying apparatus by one or more of the steps of condensing, sorption, membrane separation and pressure reduction to produce a vapour pressure <1933.2Pa (<14.5mm Hg) and wherein after calcination and reduction, the specific surface area of the cobalt in the catalyst is in the range of 5 to 30m²/g.

2. A method as claimed in Claim 1, **characterised in that** the impregnation step also includes impregnation with rhenium as a catalyst promoter component.

3. A method as claimed in any preceding Claim, **characterised in that** the dry calcining gas comprises nitrogen or air.

4. A method as claimed in any preceding Claim, **characterised in that** the support material is impregnated with an aqueous solution of cobalt nitrate hexahydrate (Co(NO₃)₂.6H₂O) by means of an incipient wetness technique whereby the catalyst contains an amount of cobalt in the range 10 to 50 wt% assuming complete reduction of cobalt.

5. A method as claimed in any preceding Claim, **characterised in that** the support material is γ-alumina or α-alumina.

6. A method as claimed in Claim 5, **characterised in that** the support material contains at least 10% by weight of a spinel compound composed of a divalent metal and aluminium.

7. A method as claimed in any preceding Claim, **characterised in that** dry calcining gas is passed over the catalyst precursor during the calcining step and has a humidity defined by a water vapour pressure of <667Pa (< 5mm Hg) and a dew point of <1.3°C, and contains less than 500 vppm of water.

## Patentansprüche

1. Verfahren zur Herstellung eines Katalysators zur Verwendung bei einer Fischer-Tropsch-Synthesereaktion, das folgende Schritte umfasst: Imprägnieren eines Katalysatorträgermaterials mit einer aktiven Cobaltkatalysatorkomponente zur Bildung einer Katalysatorvorstufe und Calcinieren und anschließendes Reduzieren der Katalysatorvorstufe, **dadurch gekennzeichnet, dass** die Katalysatorvorstufe in einer Atmosphäre eines trockenen Calcinierungsgases bei der Temperatur im Bereich von 150 °C bis 450 °C über einen Zeitraum von 1 bis 16 Stunden und einer Durchflussrate, die eine Katalysatorbelastung von mehr als 1000 h⁻¹ ergibt, calciniert wird, wobei der Wassergehalt des Calcinierungsgases in einer Gastrocknungsapparatur durch einen oder mehrere der Schritte Kondensieren, Sorption, Membrantrennung und Druckreduktion zur Erzeugung eines Dampfdrucks <1933,2 Pa (<14,5 mm Hg) verringert wird und wobei nach der Calcinierung und Reduktion die spezifische Oberfläche des Cobalts in dem Katalysator im Bereich von 5 bis 30 m²/g liegt.

2. Verfahren nach Anspruch 1, **dadurch gekennzeichnet, dass** der Imprägnierungsschritt außerdem eine Imprägnierung mit Rhenium als Katalysatorpromotorkomponente umfasst.

3. Verfahren nach einem der vorhergehenden Ansprüche, **dadurch gekennzeichnet, dass** das trockene Calcinierungsgas Stickstoff oder Luft umfasst.

4. Verfahren nach einem der vorhergehenden Ansprüche, **dadurch gekennzeichnet, dass** das Trägermaterial mit einer wässrigen Lösung von Cobaltnitrathexahydrat (Co(NO₃)₂.6H₂O) mit Hilfe einer Incipient-Wetness-Technik imprägniert wird, wodurch der Katalysator eine Cobaltmenge im Bereich von 10 bis 50 Gew.-% unter Annahme der vollständigen Reduktion von Cobalt aufweist.

5. Verfahren nach einem der vorhergehenden Ansprüche, **dadurch gekennzeichnet, dass** es sich bei dem Trägermaterial um γ-Aluminiumoxid oder α-Aluminiumoxid handelt.

6. Verfahren nach Anspruch 5, **dadurch gekennzeichnet, dass** das Trägermaterial mindestens 10 Gew.-% einer Spinellverbindung, die aus einem zweiwertigen Metall und Aluminium besteht, enthält.

7. Verfahren nach einem der vorhergehenden Ansprüche, **dadurch gekennzeichnet, dass** trockenes Calcinierungsgas während des Calcinierungsschritts über die Katalysatorvorstufe geleitet wird und eine durch einen Wasserdampfdruck von <667 Pa (<5 mm Hg) und einen Taupunkt von <1,3 °C definierte Feuchtigkeit aufweist und weniger als 500 vppm Wasser enthält.

## Revendications

1. Procédé de production d'un catalyseur pour utilisation dans une réaction de synthèse de Fischer-Tropsch, comprenant les étapes de : imprégnation d'un matériau de support de catalyseur avec un composant de catalyseur au cobalt actif pour former un précurseur de catalyseur, et calcination, puis réduction du précurseur de catalyseur, **caractérisé en ce que** le précurseur de catalyseur est calciné dans une atmosphère d'un gaz de calcination sec à une température dans la plage de 150 °C à 450 °C pendant une durée de 1 à 16 heures et à un débit produisant une vitesse spatiale horaire de gaz supérieure à 1000 h⁻¹, la teneur en eau du gaz de calcination étant réduite dans un appareil de séchage de gaz par une ou plusieurs des étapes de condensation, sorption, séparation sur membrane et réduction de pression pour produire une pression de vapeur < 1933,2 Pa (< 14,5 mmHg) et dans lequel, après calcination et réduction, la surface spécifique du cobalt dans le catalyseur est dans la plage de 5 à 30 m²/g.

2. Procédé selon la revendication 1, **caractérisé en ce que** l'étape d'imprégnation comprend en outre l'imprégnation avec du rhénium en tant que composant de promoteur de catalyseur.

3. Procédé selon l'une quelconque des revendications précédentes, **caractérisé en ce que** le gaz de calcination sec comprend de l'azote ou de l'air.

4. Procédé selon l'une quelconque des revendications précédentes, **caractérisé en ce que** le matériau de support est imprégné avec une solution aqueuse de nitrate de cobalt hexahydraté (Co(NO₃)₂.6H₂O) au moyen d'une technique d'humectation initiale de sorte que le catalyseur contienne une quantité de cobalt dans la plage de 10 à 50 % en poids en supposant une réduction totale du cobalt.

5. Procédé selon l'une quelconque des revendications précédentes, **caractérisé en ce que** le matériau de support est la γ-alumine ou la α-alumine.

6. Procédé selon la revendication 5, **caractérisé en ce que** le matériau de support contient au moins 10 % en poids d'un composé de spinelle constitué d'un métal divalent et d'aluminium.

7. Procédé selon l'une quelconque des revendications précédentes, **caractérisé en ce que** le gaz de calcination sec est passé sur le précurseur de catalyseur pendant l'étape de calcination et a une humidité définie par une pression de vapeur d'eau < 667 Pa (< 5 mmHg) et un point de rosée < 1,3 °C, et contient moins de 500 ppm en volume d'eau.
